# EUROPÄISCHE PATENTSCHRIFT

(11) **EP 2 157 913 B1**
(45) Veröffentlichungstag und Bekanntmachung des Hinweises auf die Patenterteilung: **26.01.2011**
(21) Anmeldenummer: 08735817.2
(22) Anmeldetag: 04.04.2008
(51) Int. Cl.: A61B 6/00, G06T 11/00

(54) **VERFAHREN UND VORRICHTUNG ZUR GEWINNUNG EINES VOLUMENDATENSATZES VON EINEM BEWEGTEN GEWEBE ODER ORGAN EINES PATIENTEN**
METHOD AND DEVICE FOR OBTAINING A VOLUME DATA SET OF A MOBILE TISSUE OR ORGAN OF A PATIENT
PROCÉDÉ ET DISPOSITIF PERMETTANT D'OBTENIR UN ENSEMBLE DE DONNÉES DE VOLUME D'UN TISSU MOBILE OU ORGANE D'UN PATIENT

(30) Priorität: 10.04.2007 DE 102007016902
(43) Veröffentlichungstag der Anmeldung: 03.03.2010
(73) Patentinhaber: Biosense Webster, Inc., Diamond Bar, CA 91765 (US)
(72) Erfinder: RAHN, Norbert, 91301 Forchheim (DE); BARTAL, Meir, 30900 Zichron Yaakov (IL); BOESE, Jan, 90542 Eckental (DE); GOVARI, Assaf, 34400 Haifa (IL); JOHN, Matthias, 90429 Nürnberg (DE); PREISS, Assaf, 17906 Shimshit (IL)
(74) Vertreter: Brown, George Laurence
(86) Internationale Anmeldenummer: PCT/EP2008/054076
(87) Internationale Veröffentlichungsnummer: WO 2008/122599

(56) Entgegenhaltungen:
- WO-A-2007/015181
- WO-A-2007/136967
- US-A1- 2003 152 195
- MOVASSAGHI B ET AL: "3D coronary reconstruction from calibrated motion-compensated 2D projections based on semi-automated feature point detection" PROCEEDINGS OF SPIE-THE INTERNATIONAL SOCIETY FOR OPTICAL ENGINEERING, SPIE, BELLINGHAM, WA; US, Bd. 5370, Nr. 1, 16. Februar 2004 (2004-02-16), Seiten 1943-1950, XP007904504

## Beschreibung

Die Erfindung betrifft ein Verfahren und eine Vorrichtung zur Gewinnung eines Volumendatensatzes von einem bewegten Gewebe oder Organ eines Patienten mit einem C-Bogen-Röntgengerät und einem elektromagnetischen Positionserfassungssystem.

Ein System aus einem C-Bogen-Röntgengerät und einem elektromagnetischen Positionserfassungssystem, welches Positionserfassungssystem zudem als Mappingsystem ausgebildet sein kann, kommt beispielsweise bei Punktionen, allgemeinen Katheteranwendungen oder Katheteranwendungen am Herzen eines Patienten zum Einsatz, wobei basierend auf mit dem C-Bogen-Röntgengerät gewonnenen Bildern von dem Patienten und auf mit dem elektromagnetischen Positionserfassungs- und Mappingsystem gewonnenen Positionsdaten der Punktionsnadel oder des Katheters die Punktionsnadel oder der Katheter in dem Patienten navigiert bzw. geführt wird, indem z.B. ein Abbild der Punktionsnadel oder des Katheters in die mit dem C-Bogen-Röntgengerät gewonnenen Bilder eingeblendet wird. Das elektromagnetische Positionserfassungs- und Mappingsystem und das C-Bogen-Röntgengerät bzw. das elektromagnetische Positionserfassungs- und Mappingsystem und die mit dem C-Bogen-Röntgengerät gewonnenen Bilder sind hierzu in der Regel miteinander registriert, damit ein Abbild der Punktionsnadel oder des Katheters in die Röntgenbilder eingeblendet werden kann.

Bei der Behandlung von Herzrhythmusstörungen eines Patienten durch eine so genannte Ablation wird ein Ablationskatheter beispielsweise mit Hilfe von mit dem C-Bogen-Röntgengerät gewonnenen Durchleuchtungsbildern über Venen oder Arterien in eine der Herzkammer des Patienten eingeführt und durch Hochfrequenzstrom das die Herzrhythmusstörungen hervorrufende Gewebe verödet. Voraussetzung für eine erfolgreiche Durchführung einer Katheterablation ist einerseits die genaue Ortung der Ursache der Herzrhythmusstörungen in der Herzkammer und andererseits die zielgenaue Verödung des die Herzrhythmusstörungen hervorrufenden Gewebes. Die Ortung des Gewebes erfolgt in einer elektrophysiologischen Untersuchung, bei der elektrische Potentiale mit einem in die Herzkammer eingeführten Mapping-Katheter ortsaufgelöst erfasst werden. Aus dieser elektrophysiologischen Untersuchung, dem so genannten elektro-anatomischen Mapping, werden beispielsweise 3D-Mappingdaten von der Herzkammer erhalten, die an einem Sichtgerät visualisiert werden können. Die Mapping-Funktion und die Ablationsfunktion sind im Übrigen häufig in einem Katheter vereint, so dass der Mappingkatheter gleichzeitig auch ein Ablationskatheter ist.

Ein bekanntes elektro-anatomisches 3D-Mappingverfahren, wie es z.B. mit dem CARTO-System der Fa. Biosense Webster Inc., USA, durchführbar ist, basiert auf elektromagnetischem Prinzip. Mit unter einer Patientenlagerungsvorrichtung angeordneten Transmittern werden in der Regel drei verschiedene elektromagnetische Felder geringer Intensität aufgebaut. Mittels in die Katheterspitze des Mappingkatheters integrierter elektromagnetischer Sensoren ist es möglich, die durch die Katheterbewegungen induzierten Spannungsänderungen innerhalb der elektromagnetischen Felder zu messen und mit Hilfe mathematischer Algorithmen zu jedem Zeitpunkt die Position des Mappingkatheters zu errechnen. Durch punktweises Abtasten der Kontur einer Herzkammer mit dem Mappingkatheter bei simultaner Erfassung der elektrischen Signale der Sensoren erhält man so die Mappingdaten bzw. entsteht eine elektroanatomische dreidimensionale Landkarte.

Die Führung des Ablationskatheters kann daher nicht nur mit Hilfe der bereits erwähnten Röntgendurchleuchtungsbilder, sondern auch anhand der elektro-anatomischen Mappingdaten erfolgen. Gerade die Durchleuchtungsbilder zeigen nämlich die Anatomie des Patienten, insbesondere die Anatomie des Herzens des Patienten nicht im Detail. Eine 3D-Darstellung anatomischer Details des Herzens könnte die Genauigkeit bei der Durchführung der Ablation in Bezug auf die Morphologie des Herzgewebes erhöhen, die Durchführung der Ablation beschleunigen und zu eine Reduzierung der einem Patienten während einer Ablation applizierten Röntgenstrahlendosis führen.

Insbesondere bei komplexen Fällen begrüßen es Elektrophysiologen, die Ablation anhand einer Kombination von elektrophysiologischen und morphologischen Kriterien durchführen zu können. Für die Elektrophysiologen wäre es daher hilfreich, eine kombinierte Visualisierung von mit einem Bildgebungsgerät gewonnenen 3D-Bilddaten und elektro-anatomischen 3D-Mappingdaten zur Verfügung zu haben.

Die Rekonstruktion eines Volumendatensatzes vom Herzen eines Patienten anhand von mit einem C-Bogen-Röntgengerät aufgenommenen Röntgenprojektionen erfolgt in der Regel EKGgetriggert. So ist aus der DE 10 2005 016 472 A1 ein Betriebsverfahren für eine Röntgenanlage bekannt, bei der eine Röntgenanordnung mehrmals zwischen zwei Endlagen um eine Schwenkachse verschwenkt wird. Die Röntgenanordnung wird dabei derart angesteuert, dass jeweils bei einer Vielzahl von Winkellagen zu Erfassungszeitpunkten Röntgenprojektionen eines im Bereich der Schwenkachse angeordneten, sich bewegenden Untersuchungsobjektes erfasst und einer Steuereinrichtung zugeführt werden, welche die Röntgenprojektionen und die korrespondierenden Winkellagen speichert. Die Steuereinrichtung nimmt auch ein auf das Untersuchungsobjekt bezogenes EKG-Signal entgegen und ordnet jeder gespeicherten Projektion eine mit einer Phasenlage des Untersuchungsobjektes korrespondierende Information zu. Zur Rekonstruktion eines Volumendatensatzes wählt die Steuereinrichtung diejenigen der Röntgenprojektionen aus, bei denen die Phasenlage zumindest in etwa einer Rekonstruktionsphasenlage entspricht.

Hat der Patient Herzrhythmusstörungen, ist diese Form der EKG-Triggerung jedoch häufig nicht ideal geeignet, um einen Volumendatensatz vom Herzen des Patienten zu erzeugen, der frei von Verschmierungen bzw. Bewegungsartefakten ist, welche durch die Herzrhythmusstörungen hervorgerufen sind.

Aus US-A-2003/0152195 ist ein Verfahren und eine Vorrichtung zur Erzeugung eines Volumendatensatzes von einem Objekt unter Berücksichtigung einer Bewegung des Objektes bekannt. An dem Objekt wird eine Referenzierungsbasis angeordnet, so dass mit einem Positionserfassungssystem die Positionen und die Orientierungen der Referenzierungsbasis während einer Aufnahme einer Serie von 2-D-Projektionen von dem Objekt erfassbar sind. Der Volumendatensatz des Objektes wird anhand der Serie von 2-D-Projektionen sowie basierend auf den vorzugsweise für alle 2-D-Projektionen der Serie ermittelten Abweichungen der Positionen und der Orientierungen der Referenzierungsbasis von einer festgelegten Referenzlage der Referenzierungsbasis erzeugt.

Die Referenzierungsbasis weist beispielsweise einen aus einem röntgentransparenten Material ausgebildeten Stab auf, welcher in dem Knochen des Patienten befestigt ist. An dem Stab ist an seinem vom Patienten entfernten Ende eine röntgentransparente Markerplatte angeordnet, welche mit von dem Positionserfassungssystem erfassbaren, röntgentransparenten Markern versehen ist.

Der Erfindung liegt daher die Aufgabe zugrunde, ein Verfahren und eine Vorrichtung der eingangs genannten Art anzugeben, mit denen ein möglichst hochwertiger Volumendatensatz von einem bewegten Gewebe oder Organ eines Patienten gewonnen werden kann.

Nach der Erfindung wird diese Aufgabe gelöst durch Verfahren zur Gewinnung eines Volumendatensatzes von einem bewegten Gewebe oder Organ eines Patienten mit einem C-Bogen-Röntgengerät und einem elektromagnetischen Positionserfassungssystem nach den Patentansprüchen 1, 2 und 3.

Nach dem ersten erfindungsgemäßen Verfahren ist wenigstens ein röntgenpositiver elektromagnetischer Sensor des elektromagnetischen Positionserfassungssystems oder ein mit einer röntgenpositiven Marke versehener elektromagnetischer Sensor des Positionserfassungssystems zumindest indirekt an dem Gewebe oder Organ angeordnet, wobei der mit einer Röntgenstrahlenquelle und einem Röntgenstrahlenempfänger versehende C-Bogen des C-Bogen-Röntgengeräts zur Aufnahmen einer Vielzahl von Röntgenprojektionen von dem Gewebe oder Organ des Patienten aus unterschiedlichen Projektionsrichtungen um den Patienten verstellt wird. Der röntgenpositive elektromagnetische Sensor oder der mit einer röntgenpositiven Marke versehene elektromagnetische Sensor wird in den einzelnen Röntgenprojektionen manuell oder automatisch mit einem Verfahren der Mustererkennung detektiert und es wird basierend auf denjenigen Röntgenprojektionen ein Volumendatensatz rekonstruiert, in denen der röntgenpositive elektromagnetische Sensor oder der mit einer röntgenpositiven Marke versehene elektromagnetische Sensor im Wesentlichen eine eine bestimmte Bewegungsphase des Gewebes oder Organs kennzeichnende Position einnimmt.

Der an dem sich bewegenden Gewebe oder Organ angeordnete elektromagnetische Sensor bewegt sich dem Gewebe oder Organ entsprechend und ist in jeder Röntgenprojektionsaufnahme abgebildet. Basierend auf der der Gesamtheit der Röntgenprojektionen entnehmbaren Bewegungsbahn des elektromagnetischen Sensors können eine bestimmte Position des elektromagnetischen Sensors die einer bestimmten Bewegungsphase des Gewebes oder Organs entspricht herausgegriffen und unter Berücksichtigung des jeweiligen Projektionswinkels die Röntgenprojektionen für die Rekonstruktion des Volumendatensatzes herangezogen werden, in denen der elektromagnetische Sensor die bestimmte Position wenigstens im Wesentlichen eingenommen hat, welche die interessierende bestimmte Bewegungsphase des Gewebes oder Organs kennzeichnet. Zur Festlegung der bestimmten Position des elektromagnetischen Sensors und somit zur Festlegung der bestimmten Bewegungsphase des Gewebes oder Organs kann zunächst unter Verwendung im Wesentlichen aller Röntgenprojektionen ein Volumendatensatz rekonstruiert werden, der zwar Verschmierungen aufweist, aber vor allem die Bewegungsbahn des elektromagnetischen Sensors veranschaulicht. Basierend auf dieser Bewegungsbahn können, wie bereits erwähnt, die interessierende Bewegungsphase festgelegt und diejenigen Röntgenprojektionsaufnahmen ausgewählt werden, die im Wesentlichen zu dieser Bewegungsphase aufgenommen wurden. In der Regel wird es dabei erforderlich sein, den C-Bogen des C-Bogen-Röntgengeräts mehrmals, gegebenenfalls alternierend unter Aufnahme von Röntgenprojektionen um den Patienten, insbesondere das bewegte Gewebe bzw. Organ des Patienten, zu verstellen, um genügend Röntgenprojektionen zu der Bewegungsphase des Gewebes oder Organs aus unterschiedlichen Projektionsrichtungen für die Rekonstruktion des Volumendatensatzes zur Verfügung zu haben.

Nach dem zweiten erfindungsgemäßen Verfahren werden die Positionen des elektromagnetischen Sensors mit dem elektromagnetischen Positionserfassungssystem detektiert und basierend auf denjenigen Röntgenprojektionen ein Volumendatensatz rekonstruiert, die aufgenommen worden sind, als sich der elektromagnetische Sensor im Wesentlichen an einer eine bestimmte Bewegungsphase des Gewebes oder Organs kennzeichnenden Position befunden hat. Das C-Bogen-Röntgengerät bzw. die Aufnahme der Röntgenprojektionen mit dem C-Bogen-Röntgengerät und die Bestimmung der Positionen des elektromagnetischen Sensors mit dem elektromagnetischen Positionserfassungssystem sind dabei zeitlich synchronisiert, wobei immer dann, wenn eine Röntgenprojektion aufgenommen wird, die Position des elektromagnetischen Sensors erfasst und vorzugsweise der jeweiligen Röntgenprojektion zugeordnet wird. Die Positionen des Sensors können beispielsweise bezüglich eines dem elektromagnetischen Positionserfassungssystem zugeordneten Koordinatensystems erfasst werden. Aus der Gesamtheit der Positionsinformationen des Sensors erhält man auch eine Bewegungskurve des elektromagnetischen Sensors über der Zeit, anhand der eine bestimmte Bewegungsphase des Gewebes oder Organs ausgewählt werden kann. Anschließend werden die zu dieser Bewegungsphase gehörigen Röntgenprojektionsaufnahmen für die Rekonstruktion des Volumendatensatzes herangezogen, wobei die relevanten Röntgenprojektionsaufnahmen beispielsweise durch ihre Aufnahmezeit identifizierbar sind, zu denen der elektromagnetische Sensor jeweils die die bestimmte ausgewählte Bewegungsphase kennzeichnende Position eingenommen hat.

Nach dem dritten erfindungsgemäßen Verfahren werden die Position des elektromagnetischen Sensors mit dem elektromagnetischen Positionserfassungssystem detektiert und der mit einer Röntgenstrahlenquelle und einem Röntgenstrahlenempfänger versehene C-Bogen des C-Bogen-Röntgengeräts zur Gewinnung einer Vielzahl von Röntgenprojektionen von dem Gewebe oder Organ des Patienten aus unterschiedlichen Projektionsrichtungen um den Patienten verstellt, wobei nur dann eine Röntgenprojektion für die Rekonstruktion des Volumendatensatzes aufgenommen wird, wenn sich der elektromagnetische Sensor an einer eine bestimmte Bewegungsphase kennzeichnenden Position befindet. In diesem Fall wird also die Bewegungskurve des elektromagnetischen Sensors über der Zeit mit dem elektromagnetischen Positionserfassungssystem ermittelt und basierend auf der Bewegungskurve des elektromagnetischen Sensors eine bestimmte Bewegungsphase des Gewebes oder Organs ausgewählt. Während der Verstellung des C-Bogens um das Gewebe oder Organ wird schließlich immer dann eine Röntgenprojektion ausgelöst, wenn sich der elektromagnetische Sensor wenigstens im Wesentlichen an der bestimmten Position befindet.

Auch im Falle der beiden Alternativverfahren wird der C-Bogen des C-Bogen-Röntgengeräts in der Regel mehrmals um den Patienten verstellt werden müssen, um genügend Röntgenprojektionen aus unterschiedlichen Projektionsrichtungen für die Rekonstruktion eines Volumendatensatzes zu der ausgewählten Bewegungsphase des Gewebes oder Organs zur Verfügung zu haben.

Allen erfindungsgemäßen Verfahren ist gemeinsam, dass sie hinsichtlich der Triggerung oder des Gatings der Röntgenprojektionsaufnahmen besser als die konventionellen Verfahren, insbesondere an Patienten, die an Herzrhythmusstörungen leiden, angepasst sind, da mit Hilfe des elektromagnetischen Sensors selbst kleinste Bewegungen des sich bewegenden Gewebes oder Organs erfasst, gemessen und entsprechend berücksichtigt werden können.

Nach einer Variante der Erfindung handelt es sich bei dem bewegten Gewebe oder Organ um das Herz oder die Lunge eines Patienten.

Nach einer Ausführungsform der Erfindung sind das C-Bogen-Röntgengerät und das elektromagnetische Positionserfassungssystem miteinander registriert. Unter einer Registrierung wird dabei verstanden, dass das C-Bogen-Röntgengerät und das elektromagnetische Positionserfassungssystem definiert zueinander ausgerichtet sind und Positionsänderungen des einen Gerätes oder Systems oder von Komponenten des einen Gerätes oder Systems dem anderen Gerät oder System mitgeteilt oder zur Verfügung gestellt werden.

Nach einer Variante der Erfindung umfasst die Registrierung des C-Bogen-Röntgengeräts und des elektromagnetischen Positionserfassungssystems miteinander die Ermittlung einer Koordinatentransformation zwischen einem dem C-Bogen-Röntgengerät zugeordneten Bildkoordinatensystem und einem dem Positionserfassungssystem zugeordneten Koordinatensystem anhand von wenigstens einem an der Körperoberfläche des Patienten angeordneten röntgenpositiven elektromagnetischen Körpersensor oder anhand von wenigstens einem an der Körperoberfläche des Patienten angeordneten, mit einer röntgenpositiven Marke versehenen elektromagnetischen Körpersensor des elektromagnetischen Positionserfassungssystems, der sowohl in den Röntgenprojektionen oder im Volumendatensatz als auch mit dem Positionserfassungssystem detektierbar ist. In der Regel wird die Koordinatentransformation anhand von drei oder mehr elektromagnetischen Körpersensoren ermittelt. Basierend auf der Koordinatentransformation sind insbesondere Positionen elektromagnetischer Sensoren des elektromagnetischen Positionserfassungssystems in das Bildkoordinatensystem bzw. in mit dem C-Bogen-Röntgengerät aufgenommene Bilder transformierbar.

Nach einer weiteren Ausführungsform der Erfindung sind das C-Bogen-Ronlgengerät und das Positionserfassungssystem über wenigstens eine Datenschnittstelle miteinander verbunden, über die das C-Bogen-Röntgengerät und das Positionserfassungssystem Positionsdaten miteinander austauschen können. Auf diese Weise hat das elektromagnetische Positionserfassungssystem ständig Zugriff auf Positionsinformationen des C-Bogen-Röntgengeräts, insbesondere des C-Bogens. Ebenso hat das C-Bogen-Röntgengerät stets Zugriff auf Positionsinformationen von elektromagnetischen Sensoren bzw. Positionssensoren des elektromagnetischen Positionserfassungssystems.

Eine Variante der Erfindung sieht vor, dass unter Berücksichtigung der Projektionsrichtung einer Röntgenprojektion und basierend auf der Position die der röntgenpositive elektromagnetische Sensor oder der mit einer röntgenpositiven Marke versehene elektromagnetische Sensor bei dieser Röntgenprojektion eingenommen hat, ein Abbild des röntgenpositiven elektromagnetischen Sensors oder des mit einer röntgenpositiven Marke versehenen elektromagnetischen Sensors in die jeweilige Röntgenprojektion eingeblendet wird. Diese projektivische Einblendung eines Abbildes des elektromagnetischen Sensors kann insbesondere die manuelle oder auch automatisch vornehmbare Detektion des elektromagnetischen Sensors in den Röntgenprojektionsaufnahmen erleichtern. Die Einblendung kann auch zu Kontrollzwecken hinsichtlich des zweiten und dritten erfindungsgemäßen Verfahrens verwendet werden, wozu die dabei verwendeten elektromagnetischen Sensoren röntgenpositiv oder mit einer röntgenpositiven Marke versehene sein müssen.

Nach einer Ausführungsform der Erfindung werden basierend auf den Positionen des röntgenpositiven elektromagnetischen Sensors oder des mit einer röntgenpositiven Marke versehenen elektromagnetischen Sensors und den Röntgenprojektionsaufnahmen und/oder basierend auf dem in den Röntgenprojektionsaufnahmen abgebildeten elektromagnetischen Körpersensor oder den mit einer röntgenpositiven Marke versehenen elektromagnetischen Körpersensor die Projektionsmatrizen des C-Bogen-Röntgengeräts neu ermittelt. In der Regel werden die Projektionsmatrizen des C-Bogen-Röntgengeräts in einem Kalibrierprozess vor der Inbetriebnahme des C-Bogen-Röntgengeräts mit einem Phantom ermittelt, wobei die Kenntnis der Projektionsmatrizen notwendig ist, um aus einer Vielzahl von aus unterschiedlichen Projektionsrichtungen aufgenommenen Röntgenprojektionen einen Volumendatensatz rekonstruieren zu können. Die Ermittlung der Projektionsmatrizen, die die Projektionsgeometrien des C-Bogen-Röntgengeräts enthalten, ist dabei erforderlich, da der C-Bogen für sich kein absolut steifes mechanisches System ist, sondern bei Verstellungen des C-Bogens um einen Patienten oder ein Untersuchungsobjekt mechanische Deformationen, Gravitationseffekt, Verwindungen des C-Bogens etc. auftreten. Es sind daher für jedes C-Bogen-Röntgengerät die Projektionsmatrizen in einem Kalibrierprozess zu ermitteln und für die spätere Rekonstruktion von Volumendatensätzen von Objekten abrufbar abzuspeichern. Gemäß der erwähnten Variante der Erfindung werden die Projektionsmatrizen basierend auf den Positionen des elektromagnetischen Sensors und den Röntgenprojektionsaufnahmen und/oder basierend auf dem in den Röntgenprojektionsaufnahmen abgebildeten Körpersensor während einer Patientenmessung erneut ermittelt. Dies bietet die Möglichkeit, die ursprünglich ermittelten Projektionsmatrizen zu verifizieren.

Eine Variante der Erfindung sieht daher vor, die Projektionsmatrizen des C-Bogen-Röntgengeräts durch die neu ermittelten Projektionsmatrizen bei feststellbaren signifikanten Änderungen zu korrigieren und für die Rekonstruktion eines Volumendatensatzes zu verwenden oder nur die neu ermittelten Projektionsmatrizen für die Rekonstruktion eines Volumendatensatzes zu verwenden.

Nach einer weiteren Variante der Erfindung werden mit einer EKG-Vorrichtung EKG-Signale oder mit einer Vorrichtung zur Aufnahme des Atemzyklus den Atemzyklus betreffende Daten zusätzlich aufgenommen, die als Sekundärinformationen verwendet werden können. Die EKG-Signale oder die den Atemzyklus betreffenden Signale können auch dann verwendet werden, wenn das bewegte Gewebe oder Organ über einen Zeitabschnitt einen regelmäßigen Rhythmus bzw. Zyklus zeigt. Insofern ergibt sich eine kombinierte Anwendung. Die EKG-Signale und/oder die den Atemzyklus betreffenden Daten werden dabei dem C-Bogen-Röntgengerät und/oder dem Positionserfassungssystem zur Verfügung gestellt.

Die die Vorrichtung betreffende Aufgabe wird gelöst durch eine Vorrichtung aufweisend ein C-Bogen-Röntgengerät, ein elektromagnetisches Positionserfassungssystem und wenigstens eine Recheneinrichtung, welche zur Durchführung einer der vorstehend beschriebenen Verfahren eingerichtet ist. Das elektromagnetische Positionserfassungssystem ist dabei bevorzugt an einer Patientenlagerungsvorrichtung definiert angeordnet, wobei das C-Bogen-Röntgengerät und die Patientenlagerungsvorrichtung vorzugsweise definiert relativ zueinander angeordnet sind.

Ein Ausführungsbeispiel der Erfindung ist in den beigefügten schematischen Zeichnungen dargestellt. Es zeigen:
- FIG 1: eine erfindungsgemäße Vorrichtung zur Ausführung des erfindungsgemäßen Verfahrens und
- FIG 2: eine Bewegungskurve des elektromagnetischen Sensors über der Zeit.

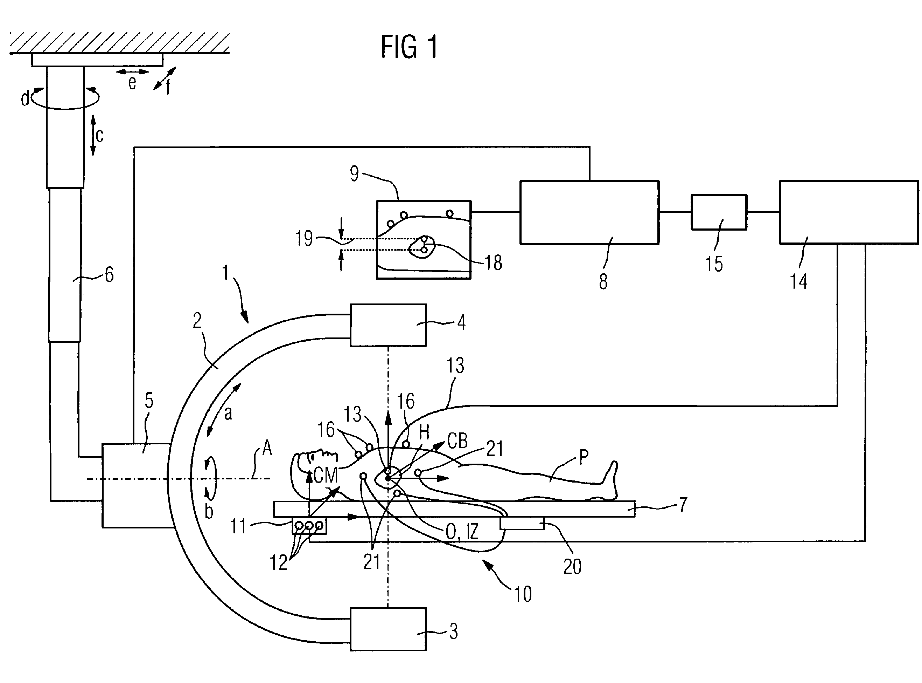

Die Vorrichtung nach FIG 1 umfasst ein C-Bogen-Röntgengerät 1 mit einem C-Bogen 2, an dem einander gegenüberliegend eine Röntgenstrahlenquelle 3 und ein Röntgenstrahlenempfänger 4 angeordnet sind. Ein Zentralstrahl eines von der Röntgenstrahlenquelle 3 ausgehenden Röntgenstrahlenbündels verläuft dabei wenigstens im Wesentlichen durch das Isozentrum IZ des C-Bogens 2 und trifft wenigstens annähernd mittig auf das Eingangsfenster des Röntgenstrahlenempfängers 4 auf. Der C-Bogen 2 ist an einer Halterung 5 um seine Orbitalachse O in die Richtungen des Doppelpfeils a verstellbar gelagert. Die Halterung 5 ist im Falle des vorliegenden Ausführungsbeispiels an einem Deckenstativ angeordnet, welches die in den Figuren mit Doppelpfeilen c, d, e und f gekennzeichneten Verstellmöglichkeiten der mit dem C-Bogen 2 versehenen Halterung 5 bietet. Außerdem ist der C-Bogen 2 mit der Halterung 5 um seine Angulationsachse A in die Richtungen des Doppelpfeils b verstellbar.

Das C-Bogen-Röntgengerät 1 weist eine Vielzahl nicht dargestellter Positionsgeber auf, von denen Positionsänderungen von Komponenten des C-Bogen-Röntgengeräts 1 erfasst werden, wobei eine jeweils aktuelle Position einer Komponente des C-Bogen-Röntgengeräts 1 im Rechner 8 des C-Bogen-Röntgengeräts 1 vorliegt. Wird beispielsweise der C-Bogen 2 um seine Orbitalachse O in eine Richtung des Doppelpfeils a verstellt, so wird dies durch Positionsgeber erfasst und die aktuelle Position des C-Bogens 2 liegt im Rechner 8 des C-Bogen-Röntgengeräts 1 vor. Ebenso verhält es sich bei Verstellungen der Halterung 5, des Deckenstativs 6 oder anderer Komponenten des C-Bogen-Röntgengeräts 1.

Mit dem C-Bogen-Röntgengerät 1 können in an sich bekannter Weise von einem auf einem Patiententisch 7 gelagerten Patienten P 2D-Röntgenprojektionen aus unterschiedlichen Projektionsrichtungen aufgenommen werden. Das C-Bogen-Röntgengerät 1 und der Patiententisch 7 sind definiert relativ zueinander angeordnet bzw. ausgerichtet und miteinander registriert. Positionsänderungen des Patiententischs 7, seien es Höhenverstellungen oder horizontale Verschwenkungen um eine nicht dargestellte vertikal verlaufende Achse, werden ebenfalls, beispielsweise über Positionsgeber, erfasst und stehen dem Rechner 8 über eine nicht dargestellte Verbindung zur Verfügung .

Das C-Bogen-Röntgengerät 1 und der mit dem Patienten P versehene Patiententisch 7 werden zur Gewinnung von 2D-Röntgenprojektionen von einem interessierenden Gewebebereich des Patienten P vorzugsweise derart relativ zueinander ausgerichtet, dass der interessierende Gewebebereich wenigstens annähernd im Isozentrum IZ des C-Bogen-Röntgengeräts 1 zu liegen kommt .

Im Falle des vorliegenden Ausführungsbeispiels soll ein Volumendatensatz vom Herzen H des Patienten P erzeugt werden. Hierzu sind mit dem C-Bogen-Röntgengerät 1 eine Vielzahl von 2D-Röntgenprojektionen vom Herzen H des Patienten P aus unterschiedlichen Projektionsrichtungen aufzunehmen, um daraus einen Volumendatensatz vom Herzen H des Patienten P rekonstruieren zu können. Die Rekonstruktion erfolgt im Fall des vorliegenden Ausführungsbeispiels durch den Rechner 8 des C-Bogen-Röntgengeräts 1, dem hierfür zuvor in einem Kalibrierprozess ermittelte Projektionsmatrizen, die die Projektionsgeometrien des C-Bogen-Röntgengeräts 1 enthalten, zur Verfügung stehen.

Da es sich beim Herzen H des Patienten P um ein bewegtes Organ handelt, muss bei der Rekonstruktion des Volumendatensatzes die Bewegung des Herzens berücksichtigt werden, um einen hochwertigen, von Bildverschmierungen freien Volumendatensatz vom Herzen, insbesondere zu einer bestimmten Herzphase erhalten zu können, aus dem 3D-Bilder vom Herzen H zu der bestimmten Herzphase des Patienten P erzeugt und auf einem Sichtgerät 9 dargestellt werden können.

Bisher werden die Bewegungen des Herzens H bzw. die Herzphasen mit einer EKG-Vorrichtung 20, an die EKG-Elektroden 21 angeschlossen sind, in einem EKG (Elektrokardiogramm) ermittelt. Die EKG-Elektroden 21 werden dabei an der Körperoberfläche des Patienten P im Bereich des Herzens H angeordnet. Anhand des EKGs können retrospektiv aus aufgenommenen 2D-Röntgenprojektionen diejenigen ausgewählt werden, die zu einer bestimmten Herzphase aufgenommen wurden, um einen hochwertigen, von Bewegungsartefakten freien Volumendatensatz vom Herzen zu dieser bestimmten Herzphase mit den ausgewählten 2D-Röntgenprojektionen rekonstruieren zu können. Die Aufnahme der 2D-Röntgenprojektionen und die Aufnahme des EKGs sind dabei miteinander synchronisiert, so dass bekannt ist, in welcher Herzphase eine 2D-Röntgenprojektion aufgenommen wurde. Die EKG-Signale stehen dem Rechner 8 des C-Bogen-Röntgengerätes 1 über eine nicht dargestellte Verbindung zur Verfügung. Dieses Verfahren zur Erzeugung eines Volumendatensatzes ist dann recht gut geeignet, wenn das Herz H des Patienten P einen verhältnismäßig gleichmäßigen Herzrhythmus und eine verhältnismäßig gleichmäßige Herzfrequenz aufweist. Im Falle eines Patienten mit Herzrhythmusstörungen hat sich dieses Vorgehen jedoch als zum Teil problematisch erwiesen.

Daher wird nach der Erfindung vorgeschlagen, am Herzen H des Patienten P, beispielsweise am Sinus coronarius, oder zumindest indirekt, d.h. nahe am Herzen H, wenigstens einen röntgenpositiven elektromagnetischen Sensor 13 anzuordnen, der zu einem elektromagnetischen Positionserfassungssystem 10 gehört. Bei dem Sensor 13 kann es sich auch um einen Katheter des Positionserfassungssystems handeln, der im Herzen, beispielsweise am Sinus coronarius platziert bzw. angeordnet ist. Das elektromagnetische Positionserfassungssystem 10 umfasst neben dem elektromagnetischen Sensor 13 eine definiert an dem Patiententisch 7 angeordnete Transmittereinheit 11 mit im Falle des vorliegenden Ausführungsbeispiels drei Transmittern 12, die jeweils ein elektromagnetisches Feld erzeugen. Die Transmittereinheit 11 und der elektromagnetische Sensor 13 sind an einer Recheneinrichtung 14 des elektromagnetischen Positionserfassungssystems 10 angeschlossen, so dass der Sensor 13 in den elektromagnetischen Feldern der Transmitter 12 detektiert und insbesondere dessen Position in eine dem elektromagnetischen Positionserfassungssystem 10 zugeordneten Koordinatensystem CM mit der Recheneinrichtung 14 ermittelbar ist. Somit können die Positionen des elektromagnetischen Sensors 13 und somit auch die Bewegungen des Herzens H erfasst werden.

Der Patient P ist im Falle des vorliegenden Ausführungsbeispiels außerdem mit drei an der Körperoberfläche des Patienten P angeordneten röntgenpositiven elektromagnetischen Körpersensoren 16 des elektromagnetischen Positionserfassungssystems 10 versehen, deren Positionen ebenfalls in dem dem elektromagnetischen Positionserfassungssystem 10 zugeordneten Koordinatensystem CM mit der Recheneinrichtung 14 ermittelt werden können. Aus Gründen der Übersichtlichkeit ist die Verbindung der Körpersensoren 16 mit der Recheneinrichtung 14 des elektromagnetischen Positionserfassungssystems 10 in der Figur nicht eingetragen.

Im Übrigen weist jeder Sensor 13 oder 16 drei elektromagnetische Empfangseinheiten auf, so dass die Positionen des jeweiligen Sensors in den drei elektromagnetischen Feldern des elektromagnetischen Positionserfassungssystems von dem Positionserfassungssystem ermittelbar sind.

Nach einem ersten Verfahren werden mit dem C-Bogen-Röntgengerät 1 unter Verstellung des C-Bogens 2 um seine Orbitalachse O um ca. 190° und damit um das Herz H des Patienten P eine Vielzahl von 2D-Röntgenprojektionen aus unterschiedlichen Projektionsrichtungen vom Herzen H aufgenommen. In diesen 2D-Röntgenprojektionen sind auch der röntgenpositive elektromagnetische Sensor 13 sowie die röntgenpositiven elektromagnetischen Körpersensoren 16 abgebildet.

Das C-Bogen-Röntgengerät 1 und das elektromagnetische Positionserfassungssystem 10 sind im Übrigen miteinander registriert, was bedeutet, dass das C-Bogen-Röntgengerät 1 und das definiert an dem Patiententisch 7 angeordnete elektromagnetische Positionserfassungssystem 10 definiert zueinander ausgerichtet sind. Das C-Bogen-Röntgengerät 1 und das elektromagnetische Positionserfassungssystem 10 sind über eine Schnittstelle 15 zwischen dem Rechner 8 und der Recheneinrichtung 14 miteinander verbunden, über die die beiden Geräte unter anderem Positionsdaten miteinander austauschen können. So stehen Positionsänderungen des C-Bogen-Röntgengeräts 1 oder von Komponenten des C-Bogen-Röntgengeräts 1 dem elektromagnetischen Positioserfassungssystem 10 und Positionsänderungen des elektromagnetischen Positionserfassungssystems 10 bzw. Positionen von Sensoren des elektromagnetischen Positionserfassungssystems 10 dem C-Bogen-Röntgengerät 1 zur Verfügung.

Anhand der Korpersensoren 16, die im Wesentlichen an Stellen der Korperoberfläche des Patienten P angeordnet sind, die wenigstens im Wesentlichen in Ruhe sind, wird im Zuge der Registrierung eine Koordinatentransformation zwischen einem dem C-Bogen-Röntgengerät 1 zugeordneten Bildkoordinatensystem CB und dem dem elektromagnetischen Positionserfassungssystem 10 zugeordneten Koordinatensystem CM ermittelt. Hierzu sind in den 2D-Rontgenprojektionen die Abbilder der Körpersensoren 16 manuell oder auch automatisch mit einem Verfahren der Mustererkennung zu lokalisieren und deren Koordinaten basierend auf den bekannten, in den Projektionsmatrizen enthaltenen Projektionsgeometrien des C-Bogen-Röntgengeräts 1 im Bildkoordinatensystem CB zu bestimmen. Des Weiteren sind die Koordinaten der elektromagnetischen Körpersensoren 16 in dem dem Positionserfassungssystem 10 zugeordneten Koordinatensystem CM zu ermitteln. Anhand der in den beiden Koordinatensystemen bekannten Koordinaten kann dann die Koordinatentransformation mit dem Rechner 8 und/oder der Recheneinrichtung 14 ermittelt werden. Auf diese Weise sind die Voraussetzungen geschaffen, Abbilder beispielsweise des Sensors 13 in 2D-Röntgenprojektionen oder in einen aus den 2D-Röntgenprojektionen rekonstruierten Volumendatensatz einzublenden.

Des Weiteren können mit Hilfe der Körpersensoren 16 unabhängig von Bewegungen des Herzens H Bewegungen des Patienten P während der Aufnahme der 2D-Röntgenprojektionen detektiert werden und eine betroffene 2D-Röntgenprojektion der Bewegung des Patienten P entsprechend korrigiert werden. Eine von einer Bewegung des Patienten P betroffene 2D-Röntgenprojektion kann dabei basierend auf einer zeitlichen Synchronisation ermittelt werden, indem die Positionen der Körpersensoren 16 zumindest während der Aufnahme der 2D-Röntgenprojektionen gleichzeitig über der Zeit erfasst werden. Da auch der Zeitpunkt der Aufnahme einer jeden 2D-Röntgenprojektion bekannt ist, kann im Falle einer Bewegung des Patienten P, welche durch eine Positionsänderung der Körpersensoren 16 registriert wird, über einen Zeitvergleich eine von der Bewegung des Patienten P betroffene 2D-Röntgenprojektion ermittelt und der Bewegung des Patienten entsprechend korrigiert werden. Die in der betroffenen 2D-Röntgenprojektion abgebildeten Gewebe des Patienten P werden dabei der Bewegung des Patienten P entsprechend derart verschoben, als ob die Bewegung nicht stattgefunden hätte.

Auf diese Weise stehen dem Rechner 8 2D-Röntgenprojektionen vom Herzen H des Patienten P zur Verfügung, die sofern sich der Patient P während der Aufnahme der 2D-Röntgenprojektionen leicht bewegt hat, ggf. diesbezüglich korrigiert sind.

Um einen Volumendatensatz von einer bestimmten Herzphase des Herzens H rekonstruieren zu können, wird der ebenfalls in den 2D-Röntgenprojektionen abgebildete Sensor 13 in den einzelnen 2D-Röntgenprojektionen manuell oder automatisch mit Hilfe eines Verfahrens der Mustererkennung detektiert. Hierzu kann zur Erleichterung der Identifikation des Sensors 13 in den einzelnen 2D-Röntgenprojektionen in jede oder auch nur in einzelne 2D-Röntgenprojektionen unter Berücksichtigung der Projektionsrichtung einer 2D-Röntgenprojektion und basierend auf der Position, die der röntgenpositive elektromagnetische Sensor 13 bei dieser 2D-Röntgenprojektion eingenommen hat, ein Abbild des Sensors 13 in die jeweilige 2D-Röntgenprojektion eingeblendet werden. Dies ist aufgrund der bekannten Koordinatentransformation und der zeitlichen Synchronisation zwischen der Aufnahme der 2D-Röntgenprojektionen und der Aufnahme der Positionen bzw. der Bewegungskurve des Sensors 13 möglich. Anschließend wird basierend auf denjenigen 2D-Röntgenprojektionen ein Volumendatensatz vom Herzen H des Patienten P rekonstruiert, in denen der röntgenpositive elektromagnetische Sensor 13 eine eine bestimmte Bewegungsphase des Herzens H kennzeichnende Position einnimmt bzw. eingenommen hat. Die Festlegung bzw. Ermittlung der bestimmten Bewegungs- oder Zyklusphase des Herzens H kann dabei anhand der Bewegungskurve des Sensors 13 über der Zeit oder auch anhand einer Hilfs-Rekonstruktion anhand von im Wesentlichen aller zur Verfügung stehender 2D-Röntgenprojektionen erfolgen. In der Hilfs-Rekonstruktion des Volumendatensatzes ist die Herz-Darstellung zwar durch Bewegungsartefakte verschmiert. Der Volumendatensatz wie er auf dem Sichtgerät 9 dargestellt werden kann, zeigt aber die Bewegungsbahn bzw. den Bewegungsverlauf 18 des Sensors 13, anhand dessen für die Rekonstruktion des Volumendatensatzes eine Herzphase ausgewählt werden kann. Im Falle des vorliegenden Ausführungsbeispiels wurde exemplarisch die oberste Extremposition 19 des Sensors 13 ausgewählt. Basierend auf dieser Auswahl werden schließlich diejenige 2D-Röntgenprojektionen identifiziert, bei deren Aufnahme der Sensor 13 im Wesentlichen die oberste Extremposition 19 eingenommen hat. Hierzu kann ein bestimmtes Intervall um die Extremposition 19 zugelassen werden, welches derart gewählt wird, dass sich praktisch keine Bewegungsartefakte im Volumendatensatz einstellen. Die identifizierten 2D-Röntgenprojektionen werden dann zur Rekonstruktion der anhand der Extremposition 19 ausgewählten Herzphase herangezogen, wodurch ein hochwertiger Volumendatensatz vom Herzen H für diese Herzphase erhalten wird.

Die Auswahl der Röntgenprojektionen für die Rekonstruktion des Volumendatensatzes erfolgt bei diesem Verfahren also Bild basiert, weshalb man auch von einem Bild basierten Gating oder Triggern sprechen kann.

Zusätzlich können basierend auf den Positionen des röntgenpositiven elektromagnetischen Sensors 13 und den 2D-Röntgenprojektionen, in denen der Sensor 13 abgebildet ist, und/oder basierend auf den in den 2D-Röntgenprojektionen abgebildeten elektromagnetischen Körpersensoren 16 die Projektionsmatrizen des C-Bogen-Röntgengeräts 1 mit dem Rechner 8 neu ermittelt werden und mit den ursprünglich in einem Kalibrierprozess ermittelten Projektionsmatrizen verglichen werden. Dies ist aufgrund der bekannten bzw. mit dem Positionserfassungssystem 10 ermittelbaren Positionen des Sensors 13 und der Körpersensoren 16 in dem dem Positionserfassungssystem zugeordneten Koordinatensystem möglich, so dass die räumlichen Positionen der Sensoren 13 und 16 bekannt sind und eine exakte Bestimmung der Parameter der Abbildungsgeometrien des C-Bogen-Röntgengerätes 1 erfolgen kann. Vorzugsweise werden insbesondere für die Neuermittlung der Projektionsmatrizen dabei mehr als die in der FIG 1 dargestellten Sensoren 13 und 16 verwendet, die sich auch in ihrer Größe voneinander unterscheiden können, um auch deren Abbildungen in 2D-Röntgenprojektionen voneinander unterscheiden zu können. Im Übrigen wird hinsichtlich der Ermittlung der Projektionsgeometrien exemplarisch auf die DE 101 40 867 B4 verwiesen, deren Verfahren zur Ermittlung von Projektionsgeometrien im vorliegenden Fall in entsprechender Weise angewendet werden kann.

Sollten sich Abweichungen zwischen neu ermittelten und ursprünglich ermittelten Projektionsmatrizen ergeben, so können entweder die ursprünglich ermittelten Projektionsmatrizen entsprechend korrigiert oder nur die neu ermittelten Projektionsmatrizen für die Rekonstruktion des Volumendatensatzes vom Herzen H verwendet werden.

Des Weiteren besteht die Möglichkeit, zusätzlich die mit der EKG-Vorrichtung synchron mit der Aufnahme der 2D-Röntgenprojektionen aufgezeichneten EKG-Signale als Sekundärinformationen zu verwenden und gegebenenfalls diese EKG-Signale zur Auswahl von 2D-Röntgenprojektionen mit heranzuziehen, wenn der Herzrhythmus des Patienten P zumindest abschnittsweise stabil ist und keine Rhythmusstörungen zeigt.

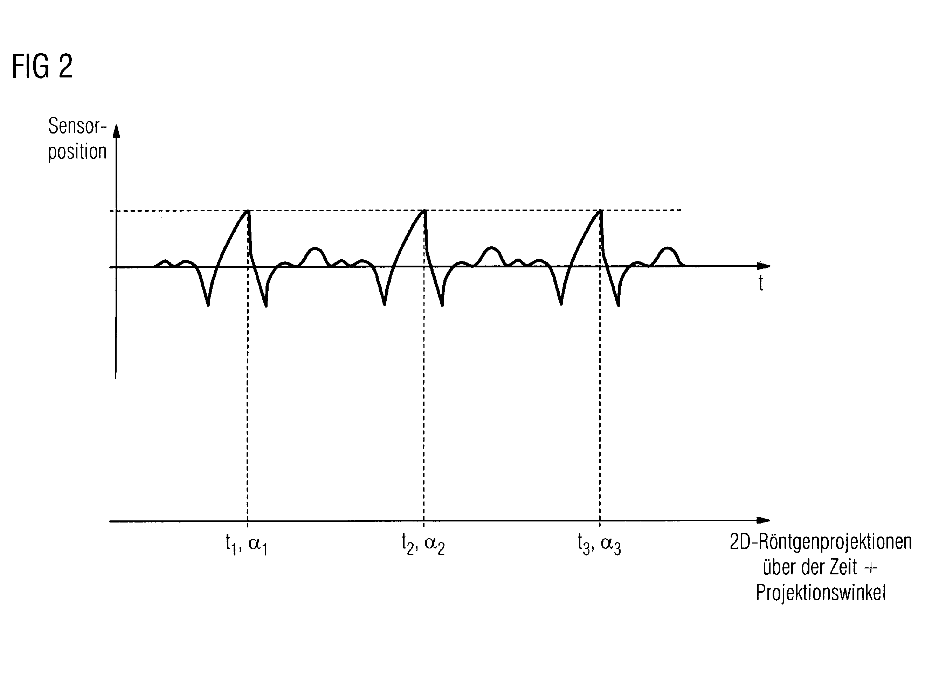

Wie bereits erwähnt, werden die Positionen des Sensors 13 während der Gewinnung der 2D-Röntgenprojektionen synchron mit der Recheneinrichtung 14 detektiert und daraus eine Bewegungskurve des Sensors 13 erzeugt, wie sie in FIG 2 dargestellt ist.

Basierend auf dieser Bewegungskurve des Sensors 13 über der Zeit kann nach einer ersten Alternative des Verfahrens eine bestimmte Herzphase des Herzens H des Patienten P ausgewählt werden und basierend auf denjenigen 2D-Röntgenprojektionen ein Volumendatensatz von der ausgewählten Herzphase des Herzens H des Patienten P rekonstruiert werden, die aufgenommen worden sind, als sich der röntgenpositive elektromagnetische Sensor 13 an einer die bestimmte Herzphase kennzeichnenden Position befunden hat. Die hierfür relevanten 2D-Röntgenprojektionen, die zu der bestimmten Position des Sensors 13 bei unterschiedlichen Projektionswinkeln aufgenommen wurden, können durch Zeitvergleich aus der Vielzahl der aufgenommenen 2D-Röntgenprojektionen retrospektiv ausgewählt werden.

Des Weiteren kann nach einer zweiten Alternative des Verfahrens zur Rekonstruktion des Volumendatensatzes so vorgegangen werden, dass basierend auf ständig ermittelten Positionen des Sensors 13 eine einer bestimmten Position des Sensors 13 entsprechende Herzphase ausgewählt wird, und immer nur dann, wenn sich der Sensor 13 an der ausgewählten Position befindet, eine 2D-Röntgenprojektion für die Rekonstruktion eines Volumendatensatzes der ausgewählten Herzphase gewonnen wird. In diesem Fall würde also immer dann, wenn der Sensor 13 eine bestimmte Position einnimmt, eine 2D-Röntgenprojektion ausgelöst. Auch in diesem Fall wird eine Bewegungskurve, wie sie in FIG 2 dargestellt ist, aufgezeichnet. Anhand der Bewegungskurve wird die Herzphase ausgewählt.

Bei allen drei Verfahren wird der C-Bogen 2 in der Regel mehrfach, beispielsweise alternierend um den Patienten P bewegt bzw. verschwenkt, um zu der ausgewählten Herzphase vom Herzen H des Patienten P 2D-Röntgenprojektionen aus unterschiedlichen Projektionsrichtungen zu gewinnen, was für die Rekonstruktion des Volumendatensatzes erforderlich ist.

Auf die beschriebene Art und Weise kann demnach ein Volumendatensatz vom Herzen H des Patienten P, der an Herzrhythmusstörungen leidet, bezüglich einer bestimmten Herzphase erzeugt werden, der beispielsweise für eine Ablationsprozedur verwendet werden kann. Das elektromagnetische Positionserfassungssystem kann dabei gleichzeitig auch ein Mappingsystem sein, um 3D-Mappingdaten vom Herzen H des Patienten P gewinnen zu können.

Die Positionen des Sensors 13 werden in solchen zeitlichen Abständen bestimmt, dass man eine Bewegungskurve des Sensors 13 über der Zeit mit hinreichender zeitlicher Auflösung für Auswahl einer bestimmten Herzphase erhält.

Im Falle der beiden zuletzt beschriebenen alternativen Verfahren muss der Sensor 13 nicht notwendigerweise röntgenpositiv oder mit einer röntgenpositiven Marke versehen sein. Ist der Sensor 13 jedoch röntgenpositiv oder mit einer röntgenpositiven Marke versehen, kann dieser Kontrollzwecken dienen. Beispielsweise lässt sich anhand des rekonstruierten Volumendatensatzes und der artefaktfreien Abbildung des Sensors 13 in dem Volumendatensatz die richtige Auswahl bzw. Aufnahmezeit der 2D-Röntgenprojektionen kontrollieren.

Im Übrigen können der im Volumendatensatz abgebildete Sensor 13 sowie die abgebildeten Körpersensoren 16 für die weitere Verwendung des Volumendatensatzes durch Bildbearbeitung aus dem Volumendatensatz entfernt werden.

Des Weiteren werden die Sensoren 13 und insbesondere die Körpersensoren 16 derart am Patienten angeordnet, dass diese interessierende Strukturen des Herzens H möglichst nicht überlagern.

Die drei erfindungsgemäßen Verfahren können im Übrigen auch beliebig miteinander kombiniert werden, um einen Volumendatensatz von einem bewegten Untersuchungsobjekt zu erzeugen.

Der Rechner 8 und die Recheneinrichtung 14 weisen entsprechende in nicht gezeigten Programmspeichern vorgehaltene Rechnerprogramme auf, um die beschriebenen Verfahren auszuführen. Dabei besteht auch die Möglichkeit nur einen Rechner vorzusehen, der sowohl das C-Bogen-Röntgengerät 1 als auch das Positionserfassungssystem 10 betreibt und die erwähnten Rechnerprogramme aufweist. Sofern zweckmäßig können auch mehr als zwei Rechner vorgesehen sein.

Die Erfindung wurde vorstehend für die Gewinnung eines Volumendatensatzes vom Herzen eines Patienten erläutert. Die Erfindung kann jedoch auch zur Gewinnung eines Volumendatensatzes von einem anderen bewegten Gewebe oder Organ, beispielsweise der Lunge eines Patienten, verwendet werden.

In diesem Fall wird anstelle einer EKG-Vorrichtung eine Vorrichtung zur Aufnahme des Atemzyklus des Patienten eingesetzt.

## Patentansprüche

1. Verfahren zur Gewinnung eines Volumendatensatzes von einem bewegten Gewebe oder Organ (H) eines Patienten (P) mit einem C-Bogen-Röntgengerät (1) und einem elektromagnetischen Positionserfassungssystem (10), bei dem
- wenigstens ein röntgenpositiver elektromagnetischer Sensor (13) oder ein mit einer röntgenpositiven Marke versehener elektromagnetischer Sensor des Positionserfassungssystem (10) zumindest indirekt an dem Gewebe oder Organ (H) angeordnet ist,
- der mit einer Röntgenstrahlenquelle (3) und einem Röntgenstrahlenempfänger (4) versehene C-Bogen (2) unter Aufnahme einer Vielzahl von Röntgenprojektionen von dem Gewebe oder Organ (H) des Patienten (P) aus unterschiedlichen Projektionsrichtungen um den Patienten (P) verstellt wird,
- der röntgenpositive elektromagnetische Sensor (13) oder der mit einer röntgenpositiven Marke versehene elektromagnetische Sensor in den einzelnen Röntgenprojektionen detektiert wird und
- bei dem basierend auf denjenigen Röntgenprojektionen ein Volumendatensatz rekonstruiert wird, in denen der röntgenpositive elektromagnetische Sensor (13) oder der mit einer röntgenpositiven Marke versehene elektromagnetische Sensor im Wesentlichen eine eine bestimmte Bewegungsphase des Gewebes oder Organs (H) kennzeichnende Position (19) einnimmt.

2. Verfahren zur Gewinnung eines Volumendatensatzes von einem bewegten Gewebe oder Organ (H) eines Patienten (P) mit einem C-Bogen-Röntgengerät (1) und einem elektromagnetischen Positionserfassungssystem (10), bei dem
- wenigstens ein elektromagnetischer Sensor (13) des Positionserfassungssystems (10) zumindest indirekt an dem Gewebe oder Organ (H) angeordnet ist,
- der mit einer Röntgenstrahlenquelle (3) und einem Röntgenstrahlenempfänger (4) versehene C-Bogen (2) unter Aufnahme einer Vielzahl von Röntgenprojektionen von dem Gewebe oder Organ (H) des Patienten (P) aus unterschiedlichen Projektionsrichtungen um den Patienten (P) verstellt wird,
- die Positionen des elektromagnetischen Sensors (13) mit dem elektromagnetischen Positionserfassungssystem (10) gleichzeitig detektiert werden und
- bei dem basierend auf denjenigen Röntgenprojektionen ein Volumendatensatz rekonstruiert wird, die aufgenommen worden sind, als sich der elektromagnetische Sensor (13) im Wesentlichen an einer eine bestimmte Bewegungsphase des Gewebes oder Organs (H) kennzeichnenden Position befunden hat.

3. Verfahren zur Gewinnung eines Volumendatensatzes von einem bewegten Gewebe oder Organ (H) eines Patienten (P) mit einem C-Bogen-Röntgengerät (1) und einem elektromagnetischen Positionserfassungssystem (10), bei dem
- wenigstens ein elektromagnetischer Sensor (13) des Positionserfassungssystems (10) zumindest indirekt an dem Gewebe oder Organ (H) angeordnet ist,
- die Positionen des elektromagnetischen Sensors (13) mit dem elektromagnetischen Positionserfassungssystem (10) detektiert werden,
- und bei dem der mit einer Röntgenstrahlenquelle (3) und einem Röntgenstrahlenempfänger (4) versehene C-Bogen (2) zur Gewinnung einer Vielzahl von Röntgenprojektionen von dem Gewebe oder Organ (H) des Patienten (P) aus unterschiedlichen Projektionsrichtungen um den Patienten (P) verstellt wird,
- wobei nur dann eine Röntgenprojektion für die Rekonstruktion des Volumendatensatzes aufgenommen wird, wenn sich der elektromagnetische Sensor (13) im Wesentlichen an einer eine bestimmte Bewegungsphase kennzeichnenden Position befindet.

4. Verfahren nach einem der Ansprüche 1 bis 3, bei dem das bewegte Gewebe oder Organ das Herz (H) oder die Lunge des Patienten (P) ist.

5. Verfahren nach einem der Ansprüche 1 bis 4, bei dem das C-Bogen-Röntgengerät (1) und das elektromagnetischen Positionserfassungssystem (10) miteinander registriert sind.

6. Verfahren nach Anspruch 5, bei dem die Registrierung des C-Bogen-Röntgengerätes (1) und des elektromagnetischen Positionserfassungssystems (10) miteinander die Ermittlung einer Koordinatentransformation zwischen einem dem C-Bogen-Röntgengerät (1) zugeordneten Bildkoordinatensystem (CB) und einem dem Positionserfassungssystem (10) zugeordneten Koordinatensystem (CM) anhand von wenigstens einem an der Körperoberfläche des Patienten (P) angeordneten röntgenpositiven elektromagnetischen Körpersensor (16) oder anhand von wenigstens einem an der Körperoberfläche des Patienten (P) angeordneten, mit einer röntgenpositiven Marke versehenen elektromagnetischen Körpersensor umfasst, der sowohl in den Röntgenprojektionen oder im Volumendatensatz als auch mit dem Positionserfassungssystem (10) detektierbar ist.

7. Verfahren nach einem der Ansprüche 1 bis 6, bei dem das C-Bogen-Röntgengerät (1) und das Positionserfassungssystem (10') über wenigstens eine Schnittstelle (15) miteinander verbunden sind, über die das C-Bogen-Röntgengerät (1) und das Positionserfassungssystem (10) Positionsdaten miteinander austauschen können.

8. Verfahren nach Anspruch 6 oder 7, bei dem anhand des Körpersensors (16) eine Bewegung des Patienten (P) bei der Gewinnung der Röntgenprojektionen detektiert und eine betroffene Röntgenprojektion der Bewegung des Patienten (P) entsprechend korrigiert wird.

9. Verfahren nach einem der Ansprüche 5 bis 8, bei dem unter Berücksichtigung der Projektionsrichtung einer Röntgenprojektion und basierend auf der Position die der röntgenpositive elektromagnetische Sensor (13) oder der mit einer röntgenpositiven Marke versehene elektromagnetische Sensor bei dieser Röntgenprojektion eingenommen hat, ein Abbild des röntgenpositiven elektromagnetischen Sensors (13) oder des mit einer röntgenpositiven Marke versehenen elektromagnetischen Sensors in die jeweilige Röntgenprojektion eingeblendet wird.

10. Verfahren nach einem der Ansprüche 1 bis 9, bei dem basierend auf den Positionen des röntgenpositiven elektromagnetischen Sensors (13) oder des mit einer röntgenpositiven Marke versehenen elektromagnetischen Sensors und den Röntgenprojektionen und/oder basierend auf dem in den Röntgenprojektionen abgebildeten elektromagnetischen Körpersensor (16) oder dem mit einer röntgenpositiven Marke versehenen elektromagnetischen Körpersensor Projektionsmatrizen des C-Bogen-Röntgengerätes (1) neu ermittelt werden.

11. Verfahren nach Anspruch 10, bei dem die ursprünglich ermittelten Projektionsmatrizen des C-Bogen-Röntgengerätes (1) durch die neu ermittelten Projektionsmatrizen bei feststellbaren Änderungen korrigiert und für die Rekonstruktion verwendet werden oder bei dem die neu ermittelten Projektionsmatrizen für die Rekonstruktion verwendet werden.

12. Verfahren nach einem der Ansprüche 1 bis 11, bei dem mit einer EKG-Vorrichtung (20) EKG-Signale oder mit einer Vorrichtung zur Aufnahme des Atemzyklus den Atemzyklus betreffenden Daten zusätzlich aufgenommen werden.

13. Verfahren nach Anspruch 12, bei dem die EKG-Signale und/oder die den Atemzyklus betreffenden Daten dem C-Bogen-Röntgengerät (1) und/oder dem Positionserfassungssystem (10) zur Verfügung gestellt werden.

14. Vorrichtung aufweisend ein C-Bogen-Röntgengerät (1), ein elektromagnetisches Positionserfassungssystem (10) und mindestens eine Recheneinrichtung (8, 14), welche zur Durchführung eines der Verfahren nach einem der Ansprüche 1 bis 13 eingerichtet ist.

## Claims

1. Method to obtain a volume data record from a moved tissue or organ (H) of a patient (P) with a C-arm x-ray machine (1) and an electromagnetic position recording system (10), in which
at least one x-ray positive electromagnetic sensor (13) or an electromagnetic sensor of the position recording system (10) provided with an x-ray positive mark is arranged at least indirectly on the tissue or organ (H),
- the C-arm (2) provided with an x-ray radiation source (3) and an x-ray radiation receiver (4) is moved around the patient (P) while recording a multitude of x-ray projections of the tissue or organ (H) of the patient (P) from various projection directions,
- the x-ray positive electromagnetic sensor (13) or the electromagnetic sensor provided with an x-ray positive mark is detected in the individual x-ray projections and
- a volume data recordis reconstructed from the volume data record based on the x-ray projections in which the x-ray positive electromagnetic sensor (13) or the electromagnetic sensor provided with an x-ray positive mark essentially records a position (19) indicating a certain movement phase of the tissue or organ (H).

2. Method to obtain a volume data record from a moved tissue or organ (H) of a patient (P) with a C-arm x-ray machine (1) and an electromagnetic position recording system (10), in which
- at least one x-ray positive electromagnetic sensor (13) of the position recording system (10) is arranged at least indirectly on the tissue or organ (H),
- the C-arm (2) provided with an x-ray radiation source (3) and an x-ray radiation receiver (4) is moved around the patient (P) while recording a multitude of x-ray projections of the tissue or organ (H) of the
patient (P) from various directions of projection,
- the positions of the electromagnetic sensor (13) are simultaneously detected by the electromagnetic position recording system (10) and
- in which a volume data record is reconstructed based on the x-ray projections recorded when the electromagnetic sensor (13) was essentially located at a position denoting a certain movement phase of the tissue or organ (H).

3. Method to obtain a volume data record from a moved tissue or organ (H) of a patient (P) with a C-arm x-ray machine (1) and an electromagnetic position recording system (10), in which
- at least one electromagnetic sensor (13) of the position recording system (10) is at least indirectly arranged on the tissue or organ (H),
- the positions of the electromagnetic sensor (13) are detected with the electromagnetic position recording system (10),
- and in which the C-arm (2) provided with an x-ray radiation source (3) and an x-ray radiation receiver (4) is moved around the patient (P) to obtain a multitude of x-ray projections of the tissue or organ (H) of the patient (P) from various directions of projection,
- wherein an x-ray projection for the reconstruction of the volume data record is recorded only when the electromagnetic sensor (13) is essentially located at a position denoting a certain movement phase.

4. Method according to any one of Claims 1 through 3, in which the moved tissue or organ is the heart (H) or the lung of the patient (P).

5. Method according to any one of Claims 1 through 4, in which the C-arm x-ray machine (1) and the electromagnetic position recording system (10) are indexed together.

6. Method according to Claim 5, in which the indexing of the C-arm x-ray machine (1) with the electromagnetic position recording system (10) comprise the determination of a transformation of coordinates between an image coordinate system (CB) attached to the C-arm x-ray machine (1) and a coordinate system (CM) attached to the position recording system (10) by means of at least one x-ray positive electromagnetic body sensor (16) arranged on the surface of the patient's body (P) or by means of at least one electromagnetic body sensor provided with an x-ray positive mark arranged on the surface of the patient's body (P), which can be detected both in the x-ray projections or in the volume data record as well as in the position recording system (10).

7. Method according to any one of Claims 1 through 6, in which the C-arm x-ray machine (1) and the position recording system (10) are connected to one another by means of at least one interface (15) by means of which the C-arm x-ray machine (1) and the position recording system (10) can be exchanged with one another.

8. Method according to Claim 6 or 7, in which a movement of the patient (P) is detected by means of the body sensor (16) as the x-ray projections are being obtained and a related x-ray projection of the movement of the patient (P) is corrected accordingly.

9. Method according to any one of Claims 5 through 8, in which, taking the projection direction of an x-ray projection into consideration, and based on the position recorded by the x-ray positive electromagnetic sensor (13) or by the electromagnetic sensor provided with an x-ray positive mark, an image of the x-ray positive electromagnetic sensor (13) or of the electromagnetic sensor provided with an x-ray positive mark is superimposed onto the respective x-ray projection.

10. Method according to any one of Claims 1 through 9, in which projection matrices of the C-arm x-ray machine (1) are newly detected, based on the position of the x-ray positive electromagnetic sensor (13) or of the electromagnetic sensor provided with an x-ray positive mark and the x-ray projections and/or based on the electromagnetic body sensor (16) displayed in the x-ray projections or in the electromagnetic body sensor provided with an x-ray positive mark.

11. Method according to Claim 10, in which if there have been identifiable changes, the originally detected projection matrices of the C-arm x-ray machine (1) are corrected by the newly detected projection matrices and used for the reconstruction, or in which the newly detected projection matrices are used for the reconstruction.

12. Method according to any one of Claims 1 through 11, in which EKG signals are recorded with an EKG device (20) or data related to the breathing cycle are also recorded with a device for recording the breathing cycle.

13. Method according to Claim 12, in which EKG signals and/or data related to the breathing cycle are provided to the C-arm x-ray machine (1) and/or to the position recording system (10).

14. Device featuring a C-arm x-ray machine (1), an electromagnetic position recording system (10) and at least one calculating device (8, 14) equipped to carry out one of the methods according to any one of Claims 1 through 13.

## Revendications

1. Procédé d'obtention d'un ensemble de données de volume d'un tissu ou organe mobile (H) d'un patient (P) avec un appareil radiographique à arceau (1) et un système de saisie de position électromagnétique (10), dans lequel
✔ au moins un capteur électromagnétique positif aux rayons X (13) ou un capteur électromagnétique doté d'une marque positive aux rayons X du système de saisie de position (10) est disposé indirectement sur le tissu ou l'organe (H),
✔ l'arceau (2) pourvu d'une source de rayons X (3) et d'un récepteur de rayons X (4) est déplacé en acquérant une multiplicité de projections de rayons X du tissu ou de l'organe (H) du patient (P) à partir de directions de projection différentes autour du patient (P),
✔ le capteur électromagnétique positif aux rayons X (13) ou le capteur électromagnétique doté d'une marque positive aux rayons X est détecté dans les projections de rayons X individuelles, et
✔ dans lequel un ensemble de données de volume est reconstruit sur la base des projections radiographiques, dans lesquelles le capteur électromagnétique positif aux rayons X (13) ou le capteur électromagnétique doté d'une marque positive aux rayons X adopte essentiellement une position (19) caractérisant une phase de mouvement déterminée du tissu ou de l'organe (H).

2. Procédé d'obtention d'un ensemble de données de volume d'un tissu ou organe mobile (H) d'un patient (P) avec un appareil radiographique à arceau (1) et un système de saisie de position électromagnétique (10), dans lequel
✔ au moins un capteur électromagnétique (13) du système de saisie de position (10) est disposé au moins indirectement sur le tissu ou l'organe (H),
✔ l'arceau (2) pourvu d'une source de rayons X (3) et d'un récepteur de rayons X (4) est déplacé en acquérant une multiplicité de projections de rayons X du tissu ou de l'organe (H) du patient (P) à partir de directions de projection différentes autour du patient (P),
✔ les positions du capteur électromagnétique (13) sont détectées en même temps que celles du système de saisie de position électromagnétique (10) et
✔ dans lequel sur la base des projections radiographiques, un ensemble de données de volume est reconstruit, qui ont été acquises lorsque le capteur électromagnétique (13) se trouvait essentiellement sur une position caractérisant une phase de mouvement déterminée du tissu ou de l'organe (H).

3. Procédé d'obtention d'un ensemble de données de volume d'un tissu ou organe mobile (H) d'un patient (P) avec un appareil radiographique à arceau (1) et un système de saisie de position électromagnétique (10), dans lequel
✔ au moins un capteur électromagnétique (13) du système de saisie de position (10) est disposé au moins indirectement sur le tissu ou l' organe (H),
✔ les positions du capteur électromagnétique (13) sont détectées avec le système de saisie de position électromagnétique (10),
✔ et dans lequel l'arceau (2) pourvu d'une source de rayons X (3) et d'un récepteur de rayons X (4) pour l'obtention d'une multiplicité de projections de rayons X du tissu ou de l'organe (H) du patient (P) est déplacé autour du patient (P) à partir de directions de projection différentes,
✔ seule une projection de rayons X étant acquise pour la reconstruction de l'ensemble de données de volume lorsque le capteur électromagnétique (13) se trouve essentiellement sur une position caractérisant une phase de mouvement déterminée.

4. Procédé selon l'une des revendications 1 à 3, dans lequel le tissu ou l'organe mobile est le coeur (H) ou le poumon du patient (P).

5. Procédé selon l'une des revendications 1 à 4, dans lequel l'appareil radiographique à arceau (1) et le système de saisie de position électromagnétique (10) sont enregistrés.

6. Procédé selon la revendication 5, dans lequel l'enregistrement de l'appareil radiographique à arceau (1) et du système de saisie de position électromagnétique (10) conjointement comprend la détermination d'une transformation de coordonnées entre un système de coordonnées d'image (CB) affecté à l'appareil radiographique à arceau (1) et un système de coordonnées (CM) affecté au système de saisie de position (10) sur la base d'au moins un capteur corporel (16) électromagnétique positif aux rayons X disposé sur la surface corporelle du patient (P) ou sur la base d'au moins un capteur corporel électromagnétique disposé sur la surface corporelle du patient (P) et doté d'une marque positive aux rayons X qui est détectable à la fois dans les projections de rayons X ou dans l'ensemble de données de volume et dans le système de saisie de position (10).

7. Procédé selon l'une des revendications 1 à 6, dans lequel l'appareil de radiographique à arceau (1) et le système de saisie de position (10) sont reliés l'un à l'autre par au moins une interface (15) par laquelle l'appareil de radiographie à arceau (1) et le système de saisie de position (10) peuvent être échangés l'un l'autre.

8. Procédé selon la revendication 6 ou 7, dans lequel, sur la base du capteur corporel (16), un mouvement du patient (P) est détecté par l'obtention des projections radiographiques et une projection radiographique concernée du mouvement du patient (P) est corrigée en conséquence.

9. Procédé selon l'une des revendications 5 à 8, dans lequel en tenant compte de la direction de projection d'une projection radiographique et sur la base de la position qu'a prise le capteur électromagnétique (13) ou le capteur électromagnétique doté d'une marque positive aux rayons X à cette projection de rayons X, une image du capteur électromagnétique positif aux rayons X (13) ou du capteur électromagnétique doté d'une marque positive aux rayons X est fondue dans la projection de rayons X respective.

10. Procédé selon l'une des revendications 1 à 9, dans lequel sur la base des positions du capteur électromagnétique positif aux rayons X (13) ou du capteur électromagnétique doté d'une marque positive aux rayons X et des projections radiographiques et/ou sur la base du capteur corporel électromagnétique (16) représenté dans les projections radiographiques ou du capteur corporel électromagnétique doté d'une marque positive aux rayons X, des matrices de projection de l'appareil radiographique à arceau (1) sont à nouveau déterminées.

11. Procédé selon la revendication 10, dans lequel les matrices de projection déterminées initialement sont corrigées par les matrices de projection déterminées nouvellement lorsque des modifications sont constatées et sont utilisées pour la reconstruction ou dans lequel les matrices de projection déterminées nouvellement sont utilisées pour la reconstruction.

12. Procédé selon l'une des revendications 1 à 11, dans lequel avec un dispositif d'ECG (20), des signaux d'ECG ou avec un dispositif d'acquisition du cycle respiratoire, des données concernant le cycle respiratoire sont également acquises.

13. Procédé selon la revendication 12, dans lequel les signaux d'ECG et/ou les données concernant le cycle respiratoire sont mis à disposition de l'appareil radiographique à arceau (1) et/ou du système de saisie de position (10).

14. Dispositif présentant un appareil radiographique à arceau (1), un système de saisie de position électromagnétique (10) et au moins un dispositif de calcul (8, 14) qui est conçu pour réaliser l'un des procédés selon l'une des revendications 1 à 13.
